# EUROPÄISCHE PATENTSCHRIFT

(11) **EP 2 155 335 B1**
(45) Veröffentlichungstag und Bekanntmachung des Hinweises auf die Patenterteilung: **01.08.2012**
(21) Anmeldenummer: 07857974.5
(22) Anmeldetag: 20.12.2007
(51) Int. Cl.: A61K 35/58, A61K 35/56, A61P 35/00, A61P 25/00

(54) **PHARMAZEUTISCHE ZUSAMMENSETZUNG, VERWENDUNG DER PHARMAZEUTISCHEN ZUSAMMENSETZUNG ZUR BEHANDLUNG EINES HIRNTUMORS, HERSTELLUNGSVERFAHREN DERSELBEN SOWIE EIN KIT OF PARTS, ENTHALTEND DIE PHARMAZEUTISCHE ZUSAMMENSETZUNG**
PHARMACEUTICAL COMPOSITION, USE OF THE PHARMACEUTICAL COMPOSITION FOR TREATING A BRAIN TUMOR, PRODUCTION PROCESS THEREOF AND A KIT OF PARTS COMPRISING THE PHARMACEUTICAL COMPOSITION
COMPOSITION PHARMACEUTIQUE, UTILISATION DE LA COMPOSITION PHARMACEUTIQUE POUR LE TRAITEMENT D'UNE TUMEUR DU CERVEAU, PROCÉDÉ DE FABRICATION DE CETTE COMPOSITION ET KIT CONTENANT LA COMPOSITION PHARMACEUTIQUE

(30) Priorität: 20.12.2006 DE 102006060344
(43) Veröffentlichungstag der Anmeldung: 24.02.2010
(73) Patentinhaber: Abitec-Angewandte Bio-Technologie GBR, 80339 Muenchen (DE)
(72) Erfinder: WEICKMANN, Dirk, 91522 Ansbach (DE)
(74) Vertreter: Isarpatent
(86) Internationale Anmeldenummer: PCT/EP2007/064355
(87) Internationale Veröffentlichungsnummer: WO 2008/074872

(56) Entgegenhaltungen:
- WO-A-01/43754
- WO-A-2005/004887
- BETTINI S: "ON THE MODE OF ACTION OF LATRODECTUS SPP. VENOM" ANNALI DELL ISTITUTO SUPERIORE DI SANITA, ROME, IT, Bd. 7, Nr. 1, 1971, Seiten 1-7, XP008072380 ISSN: 0021-2571

## Beschreibung

Die vorliegende Erfindung betrifft eine pharmazeutische Zusammensetzung, die Verwendung der pharmazeutischen Zusammensetzung zur Behandlung eines Hirntumors, ein Verfahren zur Herstellung der pharmazeutischen Zusammensetzung sowie ein Kit of parts, enthaltend die pharmazeutische Zusammensetzung.

In Deutschland treten pro Jahr etwa 7000 Fälle bösartiger Himtumore auf. Dabei handelt es sich um Astrozytome mit Malignitätsgrad II und III, sowie Glioblastome des Grades IV und Glioblastom multiforme. Der Hirntumor wird meist als Nebenbefund diagnostiziert. Oft treten aber auch Blutungen im Schädel- und Himbereich des Patienten auf, die mit Bewusstlosigkeit und/oder starken, teilweise sehr starken Schmerzen einhergehen. Ein Klinikaufenthalt wird dadurch unumgänglich.

Die Behandlung von Hirntumoren gestaltet sich als äußerst schwierig. Insbesondere bei Auftreten von Glioblastomen sind die Prognosen für den Patienten meist nicht positiv. Durch eine operative Tumorentfernung kann die Überlebenszeit um einige Monate verlängert werden und die Symptome gelindert werden. Eine dauerhafte Heilung ist jedoch höchst selten.

Derzeit werden Tumore, als die gefährlichsten und gefürchtetsten Krankheiten unserer Zeit auf eine sehr radikale und den Patienten wenig schonende Weise bekämpft. Als einfache kennzeichnende Schlagworte können hier gelten:
Stahl, Strahl und Chemotherapie
Das bedeutet einmal, dass Tumore, falls einigermaßen erreichbar, im Prinzip mit dem Skalpell herausgeschnitten, durch eine breitgefächerte Bestrahlung zerstört, oder über eine sogenannte Chemotherapie mit, auch gesunde Zellen angreifenden, aggressiven Zytostatika zerstört werden. Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt, ebenso wie die Nachteile einer Radiotherapie.

Insbesondere die operative Entfernung von Hirntumoren hat jedoch weitere Nebenwirkungen. Häufig wird eine zweite Operation des Patienten notwendig. Bei der ersten Operation und Entfernung des Hirntumors wird eine große Narbenfläche erzeugt. Dort können sich neben gesunden Gliazellen auch kranke, d. h. maligne Gliazellen anlagern. Dies sind häufig Zellen, die aus dem Tumor stammen und Metastasen erzeugen. Dadurch ist ein diffuses Rezidiv vorprogrammiert. Dieses kann nicht mehr auf herkömmlichen Behandlungswegen behandelt werden. Daher ist dieses für einen Patienten oft letal.

Nach einer Operation erfolgt üblicherweise eine Strahlentherapie und/oder eine Chemotherapie. Bei der Chemotherapie wird häufig Cisplatin in verschiedenen Kombinationen verwendet. Seit Anfang 2002 wird auch das oral einzunehmende Temozolomid verwendet. Auch IMATINIB ist ein oral einzunehmendes Zytostatikum, das Anwendung findet. Neben den bekannten Nebenwirkungen wie Erbrechen, Unwohlsein, Depressionen, durch die Chemotherapie hervorgerufenen Krebs und Nervenleiden besteht bei den oben genannten Mitteln das Problem, dass sie zur Behandlung des Hirntumors die Blut-Hirn-Schranke überwinden müssen. Die Wirkstoffe durchschreiten die Blut-Hirn-Schranke üblicherweise lediglich mit 0,4 bis 5%. Die Zusammensetzung und Funktionsweise der Blut-Hirn-Schranke sind bis heute nur in Ansätzen verstanden. Diese Barriere spielt beim Schutz des Gehirns vor schädlichen Stoffen eine Rolle, ermöglicht andererseits aber auch eine geregelte Energiezufuhr.

Unser Körper besteht aus einzelnen Organsystemen und Organen, die für ihre Funktion unterschiedliche aber konstante Bedingungen zum Beispiel an Nährstoffen, Hormonen oder Elektrolyten benötigen. Alle Organe sind durch den Blutkreislauf miteinander verbunden. Da im Blut alle Bestandteile für die Versorgung, aber auch für die Entschlackung des Körpers enthalten sind, müssen Filtersysteme dafür sorgen, dass für die einzelnen Organsysteme nur die benötigten Stoffe durchgelassen beziehungsweise teilweise zurückgehalten werden. In diesem Zusammenhang kennt man unter anderem die Blut-Gewebe-Schranke, auch als Blut-Parenchym-Schranke bezeichnet, die Blut-Leber-Schranke, die Blut-Liquor-Schranke, die Blut-Hirn-Schranke, die Liquor-Hirn-Schranke, die Blut-Nerven-Schranke, die Blut-Retina-Schranke und die Plazenta-Schranke.

Bei diesen Filtermechanismen wird durch einen so genannten Schrankeneffekt der Übertritt bestimmter Stoffe aus der Blutbahn in das jeweilige Organsystem verhindert oder eingeschränkt, wenn diese Organsysteme die Bestandteile nicht oder nur in geringerer Konzentration benötigen.

Diese "Schranken" sind keine selbständigen Organe, sondern sie werden aus einer Vielzahl von Zellen und Zellzwischenräumen gebildet, die die Blutgase, die Nährstoffe und bestimmte Chemikalien durchlassen oder als Endothelporen Makromoleküle zurückhalten. Sie können auch als Lipidmembranen in der Gefäßwand hemmend auf den Durchtritt nicht lipidlöslicher Stoffe wirken oder eine selektive Wirkung aktiver Transportprozesse in den Kapillaren bewirken. Das Gehirn und das Nervengewebe werden durch zwei Filtersysteme geschützt, die Blut-Liquor-Schranke und die Blut-Hirn-Schranke.

Das Vorhandensein und die Funktion der so genannten Blut-Hirn-Schranke ist schon seit über 100 Jahren bekannt und von Paul Ehrlich bereits 1885 im Experiment nachgewiesen worden. Innerhalb des Zentralnervensystems sind die Räume zwischen den Neuronen fast völlig durch Gliazellen und ihre Ausläufer ausgefüllt. Der gesamte Stoffwechsel der Nervenzellen geht über diese Glia- oder Endothelzellen. Sie dienen zum Einbau der Nervenzellen und Nervenfasern und zu ihrer Ernährung und Isolation. Eine Form der Gliazellen sind die Astrocyten. Sie besitzen zahlreiche Fortsätze, mit denen sie sich an der Wand der Kapillaren befestigen und eine die Kapillaren allseitig umgebende, nahezu spaltenlose Endothelauskleidung bilden. Diese Endothelzellen sind durch Verbindungselemente, die "tight junctions", verknüpft und mit einer selektiven Stoffdurchlässigkeit ausgestattet, die nur Partikel mit einem Durchmesser kleiner 20 nm passieren lässt. Auf diese Weise geht der gesamte Stoffwechsel der Nervenzellen über dieses endotheliale Geflecht, das die im Blut vorhandenen Substanzen gleich einem biologischen Filter bei Bedarf durchlässt, aber für die Gehirnfunktion schädliche Substanzen vom Nervensystem fernhält.

Dieses Endothelgeflecht und die Endothelzellen, die die Kapillaren als eine Basalmembran auskleiden, werden als Blut-Hirn-Schranke bezeichnet. Ungehindert durchgelassen werden Sauerstoff, Kohlendioxid, D-Glukose, D-Hexose, einige L-Aminosäuren und lipidlösliche Stoffe, die für die Versorgung des Gehirns notwendig sind. Ebenso werden Abbauprodukte ins Blut abgegeben. Eine gewisse Barriere stellen die Endfortsätze der Astrocyten für zahlreiche Stoffe wie bestimmte Hormone, nicht lipidlösliche, wasserlösliche und chemische Substanzen sowie Proteine dar und sichern dadurch die Aufrechterhaltung eines konstanten Milieus für die Neuronen des Nervensystems.

Das Zellgefüge der Astrozcyten ist so angeordnet, dass es eine effektive Abschottung gegen höhermolekulare Substanzen und Organismen bildet. Es ist aber auch unter Normalbedingungen nicht völlig dicht, sodass einige Partikel immer diese Schranke durchdringen können. Bei Infektionen, Traumen, Entzündungen , Vergiftungen, Hypoxidosen, Fieber und im Bereich von Tumoren werden die engen Verbindungen, die tight junctions, zwischen den Endothelzellen durch die Schwellung der Astrocythen aufgedehnt und deutlich durchlässiger für andere Stoffe. Die Änderung der Lichtungsweite erfolgt durch Quellung und Entquellung der Endothelzellen. Auch die Basalmembran der Kapillaren ist keine geschlossene Schicht. Je nach der Dichte des Fasernetzes entstehen Poren in der Membran, die aktiv am Stoffaustausch beteiligt sind.

Schon lange vor der Möglichkeit einer Antibiotikabehandlung hat man die Durchlässigkeit der Blut-Hirn-Schranke durch künstliche Fiebererzeugung ähnlich dem Vorgang bei Infektionen gesteigert und zur Therapie der Syphilis des Zentralnervensystems und zur Schockbehandlung in der Psychiatrie ausgenutzt, indem man Medikamente damit direkt an das Gehirn herangeführt hat. Nach dem Ende der Einwirkung der die Blut-Hirn-Schranke beeinflussenden Bedingungen bildet sich die zeitweise Durchlässigkeit wieder zurück.

Für die Chemotherapie von Patienten mit Hirnmetastasen solider Tumoren wird derzeit Termozolomid ein lipophiles Alkylans, klinisch erforscht. Es überwindet die Blut-Hirn-Schranke und erhöht die Strahlensensibilität von Tumoren bei simultaner Radiotherapie.

Im Gegensatz hierzu wurde aber auch versucht, eine Krebstherapie auf subtilere Weise auf Basis von Naturstoffen zu ermöglichen.

Es werden hierzu, unter anderem, viele aus giftigen Lebewesen isolierte, stark wirksame Stoffe in therapeutischen Dosen als Arzneistoffe genutzt.

Die Nutzung dieser biogenen Gifte begann schon früh in der Geschichte der Menschheit. Zur gefahrlosen Anwendung dieser Gifte waren jedoch von Anfang an gewisse Grundkenntnisse über deren Behandlung und Wirksamkeit erforderlich. Die weiter durchgeführten Versuche, die Zusammensetzung des chemischen Aufbaus biogener Gifte zu entschlüsseln, führten später zur gezielten Suche bestimmter Wirkstoffe als eigentliche Verursacher beobachteter Wirkungen.

Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem Ionenaustausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt.

Gerade in neuerer Zeit wurden aus biogenen Giften viele pharmazeutische Wirkstoffe entwickelt.

So ist aus der PCT/EP00/12902 ein pharmazeutischer Wirkstoff bekannt, bei dem gefunden wurde, dass Bestandteile der Spinnengifte von Spinnen der Familie Sicariidae zur Behandlung von Tumorerkrankungen verwendet werden können. Es werden hierbei in der Hauptsache ein Peptidtoxin aus dem Gift dieser Spinnenart, eine weitere, aus dem Gift gewonnene, antagonistisch wirkende Substanz und/oder eine Kombination dieser Bestandteile medizinisch genutzt. Vorgeschlagen wird die Verwendung von Peptidtoxinen aus beispielsweise der Gattung Sicarius zur Behandlung von Mammakarzinomen, Lungenkarzinomen, Adenokarzinomen, Leberkarzinomen sowie Melanomen. Die Behandelbarkeit von Hirntumoren wie Astrozytomen und Glioblastomen wird dagegen nicht erwähnt. Konsequenterweise wird auch das Problem der Überwindung der Blut-Hirn-Schranke nicht diskutiert.

Die in der PCT/EP00/12902 beschriebenen Wirkstoffe können zur Behandlung von Tumorerkrankungen sowie parallel bzw. unterstützend bei Tumoroperationen eingesetzt und Rest-Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet. Das Gesamtgift dieser Spinnenarten, sozusagen ein Cocktail verschiedener Substanzen, ist auf Grund seiner bereits in geringen Dosen letalen Wirkung nicht pharmazeutisch einsetzbar.

Dieser bekannte Wirkstoff wirkt jedoch in vivo in keiner Kombination bei einem Hirmtumor, insbesondere nicht bei einer besonderen Art von Hirntumor, nämlich einem Oligodendrogliom oder Oligodendrozytom. Zudem ist es für eine erfolgreiche Therapie notwendig, dass dieser Wirkstoff zu großen Teilen die Blut-Hirn-Schranke überwindet.

Eine weitere Kombination von Giftstoffen ist in der PCT/EP2006/063281 offenbart. Die darin offenbarte Kombination verschiedener Giftstoffe neutralisiert sich jedoch teilweise gegenseitig, so dass sich die erwünschte tumorzellzerstörende Wirkung nur unzureichend einstellt.

Es hat sich nämlich überraschenderweise herausgestellt, dass die Peptidtoxine von Loxosceles die Peptidtoxine von Latrodectus abbauen, so dass es zu keiner signifikanten Durchdringung der Blut-Hirn-Schranke der Loxosceles-Peptidtoxine kommt. Auf einem Elektrophoresegel zeigten sich nach einer Mischung der Peptidtoxine von Latrodectus und Loxosceles keine distinkten Banden, sondern lediglich ein Schmier, verursacht durch die Bruchstücke des Peptidverdaus der Latrodectus-Peptidtoxine. Spinnen der Gattung Loxosceles gehören wie Spinnen der Gattung Sicarius zur Familie der Sicariidae. Aufgrund der engen Verwandtschaft beider Spinnengattungen war deshalb auch für eine Mischung aus Peptidtoxinen der Gattung Sicarius mit solchen der Gattung Latrodectus keine Wirkung zu erwarten.

Es ist deshalb eine Aufgabe der vorliegenden Erfindung, eine Zusammensetzung bereitzustellen, die unter möglichst effektiver Überwindung der Blut-Hirn-Schranke eine komplikationslose Abtötung von kanzerösen Körperzellen aus dem Bereich des Himgewebes, speziell des seltenen Hirntumors Oligodendrogliom, bewirkt.

Eine andere Aufgabe der vorliegenden Erfindung ist es, ein Verfahren bereitzustellen, das die Herstellung einer pharmazeutischen Zusammensetzung zur Behandlung von Hirntumoren ermöglicht.

Es ist eine weitere Aufgabe der vorliegenden Erfindung, eine Kombination von Wirkstoffen bereitzustellen, die eine wirksame Behandlung von Hirntumoren ermöglicht.

Diese Aufgaben werden durch die Merkmale der unabhängigen Ansprüche gelöst. Bevorzugte Weiterbildungen und Ausgestaltungen der Erfindung finden sich in den Unteransprüchen.

Ein erster Aspekt der vorliegenden Erfindung betrifft daher eine pharmazeutische Zusammensetzung zur Behandlung eines Hirntumors, enthaltend in einer pharmazeutisch wirksamen Menge
a) Peptidtoxin aus Spinnen der Gattung Sicarius und/oder Mundsekret von Nattern der Gattung Elaphe; und
b) Gesamtgift oder Peptidtoxine von Spinnen der Gattung Latrodectus, mit einem Molekulargewicht von 5 kDa bis 130 kDa und der Fähigkeit zur Überwindung der Blut-Hirn-Schranke, jeweils keine Latrotoxine enthaltend.

Das Peptidtoxin der Spinnen der Gattung Sicarius und/oder das Mundsekret der Nattern der Gattung Elaphe ist in der Lage, Zellen eines Tumors zu zerstören. Durch die Kombination mit dem Gift der Spinnen der Gattung Latrodectus wird das tumorzerstörende Gift an der Blut-Hirn-Schranke vorbei in das Gehirn transportiert und kann dort den Hirntumor bekämpfen. Das Gift der Spinnen der Gattung Latrodectus wirkt somit als Durchdringungssubstanz für die Blut-Hirn-Schranke. Erst diese besondere Kombination ermöglicht daher eine Zerstörung von Tumorgewebe im Gehirn. Unerwarteterweise wurden die Peptidtoxine von Latrodectus durch die Peptidtoxine von Sicarius nicht abgebaut, so dass die Latrodectus -Peptidtoxine als Transfersubstanzen für die weiteren Tumorzellen zerstörenden Toxine die Blut-Hirn-Schranke überwinden und, in Verbindung mit den Peptidtoxinen von Sicarius und/oder Elaphe, die Tumoren im Hirnbereich, beispielsweise Oligodendrozyten, zerstören konnten.

Weiterhin wurde überraschenderweise festgestellt, dass Peptidtoxine von Elaphe ebenfalls in der Lage sind, zum einen in Verbindung mit den Peptidtoxinen aus Latrodectus die Blut-Hirn-Schranke zu durchdringen und andererseits Astrozytomzellen sowie Glioblastomzellen abzutöten. Die Peptidtoxine werden in diesem Fall aus den Mundsekreten von Elaphen gewonnen. Eine Kombination der Peptidtoxine von Elaphen-Arten mit Peptidtoxinen von Loxosceles führte dagegen zu keinem Erfolg. Die Peptidtoxine von Elaphe-Arten, gewonnen aus den Mundsekreten der Elaphen, zerstören die Peptidanteile der Loxosceles-Wirkstoffe, so dass eine Wirkung nicht mehr nachweisbar war. Dagegen war eine Kombination der Peptidtoxine aus Sicarius- und Elaphe-Arten wirksam und konnte, jeweils in Verbindung mit Latrodectus-Peptidtoxinen, die Blut-Hirn-Schranke überwinden.

Die Gewinnung der Peptidtoxine kann in an sich bekannter Weise erfolgen. Hierzu wird voll inhaltlich auf die Beschreibung der PCT/EP00/12902 verwiesen. Durch verschiedene chromatographische Verfahren, beispielsweise durch HPLC-Techniken, können die jeweiligen Gesamtgiftgemische in einzelne Fraktionen aufgetrennt und die Fraktionen dann auf ihre Wirksamkeit hin untersucht werden. Die tumorzellzerstörende Wirkung einzelner Peptidtoxinfraktionen kann in Zellkulturversuchen bestimmt werden. Die Wirksamkeit der tumorzellzerstörenden Peptidtoxinfraktionen in Verbindung mit der Blut-Him-Schranken-Durchdringungssubstanz aus Latrodectus wird dann in an sich bekannten Tiermodellen bestimmt. Beispiele hierfür sind in der PCT/EP00/12902 angegeben, auf die hier voll inhaltlich Bezug genommen wird und deren Inhalt in vollem Umfang in die vorliegende Anmeldung mit aufgenommen wird. Wie in der PCT/EP00/12902 anhand von Sicarius-Peptidtoxinen beschrieben, kann der Fachmann auch für die Peptidtoxine aus den Mundsekreten von Elaphe und aus dem Gesamtgiftcocktail von Spinnen der Gattung Latrodectus die wirksamen Bestandteile isolieren und wie oben dargestellt testen. Hierzu wird auch auf die PCT/EP2006/063281 verwiesen, deren Inhalt ebenfalls in vollem Umfang in die vorliegende Anmeldung mitaufgenommen wird und auf die die Anmelderin sich hier bezieht. Diese Anmeldung zeigt die Verwendung von Peptidtoxinen aus Latrodectus, welche in der Lage sind, die Blut-Hirn-Schranke zu durchdringen und gleichzeitig die Befähigung aufweisen, andere Peptidtoxine, nämlich die aus Sicarius und Elaphe, durch die Blut-Hirn-Schranke hindurchdringen zu lassen.

Bei Latrodectus kann, alternativ zu den Peptidtoxinfraktionen, auch das Gesamtgift der Spinnen eingesetzt werden. In allen Fällen ist es allerdings wichtig, dass die Latrotoxine aus dem Giftcocktail weitgehend entfernt werden, um die pharmazeutische Zusammensetzung, welche das Gesamtgift oder Teile des Giftes von Spinnen der Gattung Latrodectus als Mittel zur Überwindung der Blut-Hirn-Schranke enthält, für den Patienten gefahrlos anwendbar zu machen. Zur Zeit sind die Latrotoxine alpha und gamma bekannt. Diese sind zumindest in einem solchen Anteil zu entfernen, dass toxische Nabenwirkungen bei der Verabreichung an den Patienten auszuschließen sind. Bevorzugt werden nur diejenigen Fraktionen aus dem Gesamtgiftcocktail von Latrodectus in der pharmazeutischen Zusammensetzung eingesetzt, welche die Wirkstoffe enthalten, die zur Passage der Blut-Hirn-Schranke befähigt sind bzw. es den Antitumor-Wirkstoffen aus Sicarius und Elaphe ermöglichen, diese Barriere zu überwinden.

Gemäß einer bevorzugten Weiterbildung der vorliegenden Erfindung enthält die pharmazeutische Zusammensetzung zusätzlich inaktivierten Parapoxvirus ovis, bevorzugt des Stammes D1701 Hierbei handelt es sich um inaktivierten Parapoxvirus ovis des Stammes D1701, dem zusätzlich Polygeline und Aqua ad injectionem zugesetzt wurde.

Gemäß einer weiteren bevorzugten Weiterbildung der vorliegenden Erfindung werden die Spinnen der Gatten Sicarius ausgewählt aus der Gruppe, bestehend aus den Arten Sicarius oweni, Sicarius testaceus, Sicarius hahni, Sicarius albospinosus, Sicarius argentinensis, Sicarius brasiliensis, Sicarius terrosus und Sicarius terrosus formae.

Die Spinnen der Gattungen Sicarius und Latrodectus werden bevorzugt wie folgt gemolken, um den Gesamtgiftcocktail der Spinnen zu erhalten:

Die Spinnen werden mechanisch ohne jegliche elektrische Stimulation der Giftdrüsen bei Temperaturen zwischen 21 und 27°C gemolken. Der Melkvorgang passiert in einem sterilen Raum unter einer Stereolupe; unter der Lupe ist zu beobachten, dass die Spinne in die Kapillare beißt; wichtig ist, dass sie sich nicht erbricht, da die Verdauungsenzyme die Peptidtoxine zerstören würden. Der herbei gewonnene Gesamtgiftcocktail wird über ein säulenchromatografisches und/oder gängige andere, an sich bekannte Verfahren in seine einzelnen Bestandteile zerlegt.

Im Beispiel 2 wird die Gewinnung von Sicaris-Peptidtoxin näher beschrieben. Die Einzelheiten zur Gewinnung von Peptidtoxinen aus Sicarius, die ebenfalls auf die Gewinnung von Peptidtoxinen aus Latrodectus und Elaphe anwendbar sind, sind in der PCT/EP00/12902 angegeben, so dass der Fachmann anhand dieses Standes der Technik problemlos die wirksamen Fraktionen der Spinnengifte erhalten kann.

Gemäß einer anderen bevorzugten Weiterbildung der vorliegenden Erfindung ist das Peptidtoxin aus Spinnen der Gattung Sicarius das HT1 und/oder das HT2 Peptidtoxin. Im Beispiel 2 ist die Herstellung dieser Peptidtoxine näher beschrieben.

Gemäß einer anderen bevorzugten Weiterbildung der vorliegenden Erfindung enthält die Zusammensetzung zusätzlich Lachesis D6. Das Lachesis D6 (Lachesis bedeutet Grubenotter) der DHU (Deutsche Homöopathische Union) wird bevorzugt als Lösungsmittel für das Sicarius-Peptidtoxin verwendet. Insbesondere bei der Herstellung der Zusammensetzung kann Lachesis D6 vorteilhaft Anwendung finden.

Gemäß einer anderen bevorzugten Weiterbildung der vorliegenden Erfindung sind die Spinnen der Gattung Latrodectus ausgewählt aus der Gruppe, bestehend aus den Arten Latrodectus bishopi, Latrodectus curacaviensis, Latrodectus dahli, Latrodectus erythromelas, Latrodectus hasselti, Latrodectus katipo, Latrodectus menavodi, Latrodectus rhodesiensis, Latrodectus revivensis, Latrodectus schuchi, Latrodectus tredecimguttatus, Latrodectus variolus, Latrodectus hesperus, Latrodectus corallinus, Latrodectus geometricus und Latrodectus obscurior.

Gemäß einer besonders bevorzugten Weiterbildung der vorliegenden Erfindung sind die Spinnen der Gattung Latrodectus bevorzugt die Arten Latrodecuts geometricus und Latrodectus obscurior.

Auch die Mischungen unterschiedlicher Gifte der Gattungen Latrodectus oder Sicarius kann gemäß einer anderen Weiterbildung der vorliegenden Erfindung erfolgen. Die Mischung kann dabei unterschiedliche Arten der Gattung Sicarius oder Latrodectus betreffen oder auch Mischungen verschiedener Populationen einer Art.

Ein zweiter Aspekt der vorliegenden Erfindung betrifft die Verwendung einer pharmazeutischen Zusammensetzung, wie sie oben beschrieben ist, zur Herstellung eines Medikaments zur Behandlung von Hirntumoren und Glioblastomen, insbesondere zur Behandlung eines Oligodendrogliom.

Durch die erfindungsgemäße Kombination von Gift oder Teilen des Gifts von Tieren der Gattung Sicarius oder eines Mundsekrets der Gattung Elaphe mit einem Gift oder Teilen des Giftes von Tieren der Gattung Latrodectus wird die Blut-Hirn-Schranke überwunden. Das Gift der Latrodectus-Tiere dient dabei als Durchdringungssubstanz, die ein Durchdringen der Schranke ermöglicht.

Ein anderer Aspekt der vorliegenden Erfindung betrifft ein Verfahren zum Herstellen einer Zusammensetzung mit den Schritten:
Bereitstellen einer isotonischen (0,9%-igen) Natriumchloridlösung mit Sicarius-Peptidtoxin;
- wahlweise Zugabe eines weiteren Sicarius-Peptidtoxins zu der Natriumchloridlösung; und
- Zugabe eines Gesamtgiftes oder von Peptidtoxinen von Spinnen der Gattung Latrodectus mit einem Molekulargewicht von 5 kDa bis 130 kDa und der Fähigkeit zur Durchdringung der Blut-Hirn-Schranke, jeweils keine Latrotoxine enthaltend;
- Vermischen der Bestandteile, insbesondere durch Schütteln der Mischung.

Gemäß einer bevorzugten Weiterbildung dieses Aspektes wird zusätzlich vor dem Vermischen ein Immunmodulator zugegeben, bevorzugt Parapoxvirus ovis, insbesondere bevorzugt vom Stamm D1701.

Gemäß einer anderen bevorzugten Weiterbildung dieses Aspektes ist mindestens eines der Sicarius-Peptidtoxine in Lachesis D6 gelöst, bevor es zur NaCl-Lösung zugegeben wird, bevorzugt beide Sicarius-Peptidtoxine.

Gemäß einer anderen bevorzugten Weiterbildung dieses Aspektes ist das erste Sicarius-Peptidtoxin ein Sicarius-Peptidtoxin HT1 und/oder das zweite Sicarius-Peptidtoxin ein Sicarius-Peptidtoxin HT2.

Weiterhin bevorzugt werden in der erfindungsgemäßen pharmazeutischen Zubereitung Phospholipasen aus Sicarius-Arten als Durchdringungssubstanzen eingesetzt, welche die Aufnahme der Peptidtoxine in die Tumorzelle erleichtern. Derartige Substanzen und ihre Gewinnung werden beispielsweise in der PCT/EP00/12902 beschrieben.

Ein weiterer Aspekt der vorliegenden Erfindung betrifft ein Kit of parts zur Behandlung von Gehirntumoren und Glioblastomen, umfassend eine pharmazeutische Zusammensetzung wie oben beschrieben und einen Extrakt aus Weihrauch und/oder Bambus. Insbesondere bevorzugt wird hier der Extrakt aus Weihrauch- und/oder Bambusblättern verwendet.

Die Hauptbestandteile des Weihrauchs sind Harze, in denen Boswelliasäuren und ätherische Öle enthalten sind. Neben den Boswelliasäuren, die zur Gruppe der pentacyclischen Triterpene gehören, findet man auch ein tetracyclisches Triterpen, die Tirucallsäure, in den Harzen.

Bevorzugt wird in der vorliegenden Erfindung das Präparat H15 Ayurmedica verwendet. Dabei handelt es sich um Tabletten, die jeweils 400 mg standardisierten Trockenextrakt aus Boswellia cerata enthalten. Die Dosierung wird je nach Bedarf angepasst. Sie kann sehr breit variieren und liegt üblicherweise im Bereich von 3 Tabletten pro Woche bis hin zu 25 Tabletten pro Tag, bevorzugt 10 Tabletten pro Woche bis 20 Tabletten pro Tag.

Der Weihrauchextrakt wird als Alternative zu Cortison verwendet, um Ödeme im Hirn bei der Behandlung mit der pharmazeutischen Zusammensetzung gemäß der vorliegenden Erfindung zu verhindern. Ödeme treten vermehrt bei der Behandlung von Hirntumoren auf, insbesondere wenn diese schnell zerstört werden, d.h., wenn sie schnell abnehmen.

Im folgenden werden die unterschiedlichen Giftstoffe detailliert erläutert.

Die Tumorzellen werden durch Peptidtoxine, die aus Gift von Tieren der Gattung Sicarus, Sechsaugenkrabbenspinne, erhalten werden können, zerstört. Bevorzugt werden die Arten Sicarius oweni, Sicarius testaceus, Sicarius hahni, Sicarius albospinosus, Sicarius argentinensis, Sicarius brasiliensis, Sicarius terrosus und Sicarius terrosus formae zur Gewinnung des Giftes eingesetzt. Bevorzugter wird das Peptidtoxin gewonnen aus den Arten Sicarius oweni, Sicarius testaceus, Sicarius argentinensis, Sicarius terrosus und Sicarius terrosus formae, wobei die beiden letztgenannten Arten insbesondere bevorzugt sind.

Die Peptidtoxine können durch bekannte Trennmethoden, wie beispielsweise Gel-Elektrophorese oder Chromatographie, insbesondere Säulenchromatographie, gewonnen werden. Die Gewinnung des Roh-Giftcocktails der Spinnen ist unten beschrieben.

Alternativ oder zusätzlich kann das Mundsekret von Nattern der Gattung Elaphe verwendet werden. Das Mundsekret wird gewonnen, indem der Natter ein Wattestäbchen als Beuteattrappe hingehalten wird, in das die Natter anschließend beißen kann. Das an dem Wattestäbchen anhaftende Mundsekret wird mittels eines Lösungsmittels, wie beispielsweise Lachesis D6 oder 0,9%-ige NaCl-Lösung, von dem Wattestäbchen gelöst. Die resultierende Lösung kann anschließend noch durch übliche Verfahren eingeengt werden.

Für die Überwindung der Blut-Hirn-Schranke werden bevorzugt Wirkstoffe aus dem Gift von Spinnen der Art Latrodectus ("schwarze Witwen") gewonnen. Diese gehören zur Gattung der Kugelspinnen (Theridiidae). Kennzeichnend für diese Tiere ist der große Hinterleib, der an eine Kugel erinnert. Der Vorderkörper ist im Verhältnis sehr klein. Die Männchen erreichen ein Länge ohne Beine bis zu 5 mm, die Weibchen immerhin 10 bis 18 mm. Diese Tiere haben eine bräunliche bis lackschwarze Färbung. Fast immer sind rote, orange oder gelbliche Zeichnungen auf dem Hinterkörper zu erkennen. Sie sind in den unterschiedlichsten Gebieten zu finden, zum Teil kommen sie auch in den Städten vor. Der Wald wird meist nicht als Lebensraum angenommen.

Es werden bevorzugt folgende Arten verwendet:
a) Latrodectus bishopi
b) Latrodectus curacaviensis
c) Latrodectus dahli
d) Latrodectus erythromelas
e) Latrodectus hasselti
f) Latrodectus katipo
g) Latrodectus menavodi
h) Latrodectus rhodesiensis
i) Latrodectus revivensis
j) Latrodectus schuchi
k) Latrodectus tredecimguttatus
l) Latrodectus variolus
m) Latrodectus hesperus
n) Latrodectus corallinus

Die beiden letztgenannten Arten werden insbesondere bevorzugt zur Gewinnung der Peptidsubstanzen zur Überwindung der Blut-Hirn-Schranke eingesetzt, wenn Kinder behandelt werden sollen.

Insbesondere bevorzugt bei der Zusammensetzung gemäß der vorliegenden Erfindung sind die Arten Latrodectus geometricus und Latrodectus obscurior.

Die Schwarze Witwe, wie sie in der Homöopathie verwendet wird, kommt vorwiegend in Amerika entlang der Pazifikküste bis nach Kanada vor. Das homöopathisch aufbereitete Gift der Schwarzen Witwe ist ein wichtiges Mittel bei Angina pectoris. Diese Spinne hält sich normalerweise von menschlichen Ansiedelungen fern und lebt in Bodennähe zwischen Steinen und Gestrüpp. Doch wo man eine Spinne findet sind meist noch mehrere Exemplare in der direkten Umgebung.

Latrodectus bishopi kommt im zentralen und südlichen Florida vor.

Latrodectus curacaviensis ist in den gemäßigten Zonen Nord- und Südamerikas sowie auf den niederländischen Antillen beheimatet. Die Weibchen sind am ganzen Körper glänzend schwarz, nur auf dem Abdomen haben sie rote bis orange auffällige Zeichnungen. Das sagenumwobene schreckliche Gift ist für einen Menschen zwar gefährlich, jedoch nur in ganz seltenen Fällen tödlich.

Latrodectus dahli wird im Iran gefunden.

Latrodectus erythromelas findet sich in Sri Lanka.

Latrodectus hasselti ist von Indien bis Australien und Neuseeland anzutreffen und wird oft in Kellern, Garagen, Schuppen oder außen liegenden WC-Häusern gefunden. Diese Spinnen leben auch gerne unter Stapeln aus Holz und anderen Baumaterialien oder auch Unrathaufen.

Latrodectus katipo ist Neuseelands einzige giftige Spinne. Sie lebt in den meisten Küstengegenden Neuseelands, außer im äußersten Süden. Sie findet sich in Bodennähe in Gräsern und Treibholz. Der Biss der Katipo kann zwar tödlich sein, das Gegengift wirkt jedoch noch innerhalb von drei Tagen.

Latrodectus menavodi ist in Madagaskar und auf den Seychellen zu finden.

Latrodectus rhodesiensis ist im südlichen Afrika beheimatet.

Latrodectus revivensis ist in Israel, vor allem im Negev, beheimatet.

Latrodectus schuchi (neuerdings Latrodectus lilianae genannt) lebt in Europa nur in Spanien, und zwar in Aragonien.

Latrodectus tredezimguttatus ist in Südeuropa sehr oft in Getreidefeldern zu finden, vor allem im Mittelmeergebiet, zum Teil in Asien und Afrika. Das Aussehen dieser Spinne ist bei einem völlig schwarz glänzenden Körper durch 13 rote Flecken auf dem Hinterleib charakterisiert. Häufig findet man die unregelmäßig gebauten Netze in der Nähe des Erdbodens. Da zu dem Netz immer wieder neuere Fäden eingesponnen werden, ist es sehr stabil. Das Zentrum des Netzes bildet eine Einstülpung mit der Form eines Korbes und ist meist unter einem Gegenstand wie einem Stein, einem Ast oder auch in einer Mauerritze zu finden.

Latrodectus variolus wird zum Beispiel in Amerika in Michigan gefunden. Weibliche Exemplare haben einen roten Punkt auf der Unterseite ihres Hinterleibs, männliche weisen diesen nicht auf, tragen aber gelbe und rote Bänder auf dem Rücken.

Das Latrodectus-Gift besteht aus 6 bis 7 verschiedenen Proteinen mit Molekulargewichten von 5 kDa bis 130 kDa, von denen das Latrotoxin ein höchst wirksames Neurotoxin ist. Es wird hierbei der Giftcocktail oder Teile des Giftcocktails der unter a) bis n) genannten Latrodectus-Arten verwendet und insbesondere die Arten Latrodectus geometricus und Latrodectus obscurior. Die unter m) und n) genannten Latrodectus-Arten sind auch bei Kindern bis zum Alter von etwa 12 Jahren einsetzbar.

Die erfindungsgemäß für die pharmazeutische Zusammensetzung verwendeten Substanzen können auf natürlichem Weg von den Tieren gewonnen werden. Diese Gifte wurden ursprünglich zum Beutefang und zur Vorverdauung tierischen Proteins entwickelt. Diese natürliche Wirkweise kann durch eine funktionserhaltende, schonende Gewinnung des Giftgrundstoffes (z.B. durch manuelles Melken) erhalten werden.

Im Gegensatz zu herkömmlichen Melkweisen von Arthropoden mittels eines elektrischen Verfahrens (Weickmann D. (1991): Haltung und Giftigkeit von Sicariidae. Arachnologischer Anzeiger 16, S. 12-13; und ferner Weickmann D., Burda R. (1994): Electrophoresis of scorpion venoms. Electrophoresis Forum 1994, Abstracts, Technische Universität München), bei denen den Tieren das Gift über einen elektrischen Impuls, der bei den Tieren die Kontraktion der Giftdrüsen auslöst, entzogen wird (die Tiere sind hierbei bevorzugt unterkühlt), wird gemäß der vorliegenden Erfindung der Giftcocktail über ein manuelles Verfahren erhalten, bei dem die Tiere über die Ausnutzung ihres natürlichen Abwehrverhaltens zur Abgabe ihres Giftes stimuliert werden.

Gemäß einer Ausführungsform der Erfindung ist eine manuelle Melkweise der Spinnen vorgesehen. Dadurch werden echte, unverfälschte native Gifte erhalten, während im Gegensatz dazu, zum Beispiel bei der elektrischen Melkweise durch Elektronenfluss umstrukturierte Substanzen bzw. Moleküle erhalten werden, die in ihrer Wirkweise geändert sein können, oder auch Substanzen in den Giften vorhanden sein können, die das Tier sonst nicht abgeben würde. Diese Substanzen können, müssen aber nicht zwingend die Effizienz der im Giftcocktail enthaltenen medizinisch wirksamen Verbindung negativ beeinflussen. Standardmäßig kann eine Analyse und/oder Qualitätskontrolle des Rohgiftgemisches über elektrophoretische Verfahren erfolgen.

Zur manuellen Melkung werden bevorzugt subadulte Weibchen der oben genannten Arten und Gattungen mit den Fingern einer Hand in Rückenlage fixiert und durch Berühren mit der stumpfen Seite einer auf eine sterile Spritze aufgesetzten sterilen Kanüle an den Chelizeren zur Abgabe des Giftes stimuliert. Die Raumtemperatur beträgt meist etwa 21 bis 27 Grad, die Luftfeuchtigkeit 50 % bis 70 %. Die Tageszeit spielt keine Rolle.

Dabei ist es bevorzugt, dass die Stimulationszeit nicht länger als 90 Sekunden dauert, da sonst das betreffende Tier einem unnötigen Stress ausgesetzt würde. Nach Erscheinen des Gifttropfens an den Giftklauen wird dieser mit der Spritze über die Kanüle aufgezogen. Für jedes Tier wird eine neue Spritze mit neuer Kanüle verwendet. Anschließend wird die Kanüle mit der Kanülenschutzhülle wieder verschlossen. Die verschlossene Spritze samt aufgezogenem Gift wird direkt anschließend in einen Exsikator verbracht.

Es ist auch möglich, die beschriebenen Wirkstoffe, die in den Giften der unterschiedlichen Tiere enthalten sind, chemisch-synthetisch oder durch gentechnologische Methoden in rekombinierter Form herzustellen. Wie bei chemischen Substanzen üblich, umfasst die vorliegende Erfindung auch Derivate und Salze der erfindungsgemäß bereitgestellten Substanzen. Beispielsweise kann das Peptidtoxin ein oder mehrere, Substitutionen und/oder Deletionen von Aminosäuren umfassen, wobei natürlich sichergestellt sein muss, dass die erfindungsgemäße medizinische Wirkung erhalten bleibt. Die Gewinnung des beschriebenen Wirkstoffs erfolgt durch in der chemischen Verfahrenstechnik übliche Methoden.

Die Wirkstoffe der vorliegenden Erfindung werden vorzugsweise in Form einer solchen pharmazeutischen Zusammensetzung verwendet, in der sie mit geeigneten Trägem oder Trägerstoffen in Dosen vermischt werden, so daß die Erkrankung behandelt oder zumindest gelindert wird. Eine derartige Zusammensetzung kann (zusätzlich zu den Wirkstoffen und dem Träger) Verdünnungsmittel, Füllmaterialien, Salze, Puffer, Stabilisatoren, Solubilisierungsmittel und andere Materialien enthalten, die in der Technik wohlbekannt sind. Der Begriff "pharmazeutisch verträglich" ist als ein nichttoxisches Material definiert, das die Wirksamkeit der biologischen Aktivität des aktiven Inhaltsstoffes bzw. Wirkstoffes nicht stört. Die Auswahl des Trägers hängt vom Verabreichungsweg ab.

Die pharmazeutische Zusammensetzung kann zusätzlich weitere Mittel enthalten, die die Aktivität des Wirkstoffes steigern oder dessen Aktivität oder Verwendung bei der Behandlung ergänzen. Derartige zusätzliche Faktoren und/oder Mittel können in der pharmazeutischen Zusammensetzung enthalten sein, um eine synergistische Wirkung zu erzielen oder um Nebenwirkungen bzw. unerwünschte Wirkungen zu minimieren.

Techniken zur Formulierung bzw. Zubereitung und Verabreichung der Verbindungen der vorliegenden Anmeldung sind in "Remington's Pharmaceutical Sciences", Mack Publishing Co., Easton, PA, letzte Ausgabe, zu finden. Eine therapeutisch wirksame Dosis bezieht sich ferner auf eine Menge der Verbindung, die ausreicht, um eine Verbesserung der Symptome zu erreichen, beispielsweise eine Behandlung, Heilung, Prävention oder Verbesserung derartiger Zustände. Geeignete Verabreichungswege können beispielsweise orale, rektale, transmucosale oder intestinale Verabreichung und parenterale Verabreichung einschließen, einschließlich intramuskulärer, subkutaner, intramedulärer Injektionen ebenso wie intrathekaler, direkt intraventrikulärer, intravenöser, intraperitonealer oder intranasaler Injektionen. Die subkutane oder intramuskuläre Verabreichung an einen Patienten wird bevorzugt.

Verabreicht werden 1-5 ml, bevorzugt 2 - 4 ml der Zusammensetzung im Abstand von etwa 1-4 Tagen. Die Menge an verabreichter Zusammensetzung kann jedoch durch einen Fachmann entsprechend angepasst werden. Die Anpassung kann auch während der Behandlung erfolgen.

Bei den herkömmlichen Behandlungsmethoden bilden sich sowohl beim Primärtumor als auch beim Sekundär- oder Rezidivtumor gerne Ödeme, welche verstärkte Druckschmerzen auslösen. Gegen die Ödembildung wird häufig Cortison, oft auch sehr hoch dosiert, verwendet. Cortisone können jedoch ebenfalls Tumore auslösen. Zudem kann Cortison in seiner Wirkweise stark abhängig machen, so dass beim Absetzen meist ein langsames Ausschleichen in vielen, sich reduzierenden Teildosen notwendig ist.

Die Erfinder der vorliegenden Erfindung haben zudem festgestellt, dass die erfindungsgemäßen Zusammensetzungen bei ihrer Durchgängigkeit ins Gehirn durch Cortisone gestört werden. Überraschenderweise haben die Erfinder jedoch festgestellt, dass als vollwertiger Ersatz gegen die Ödembildung Weihrauch- und/oder Bambusextrakt verwendet werden kann.

Wie bereits oben erwähnt, kommen bevorzugt H15 Präparate als Weihrauchextrakt in Betracht. Als Bambusextrakt, insbesondere der Extrakt von Bambusblättern in Betracht. Es können jedoch auch direkt getrocknete Weihrauchblätter und/oder Bambusblätter in Kapselform in Kombination mit der pharmazeutischen Zusammensetzung gemäß der vorliegenden Erfindung verabreicht werden.

Im folgenden wird anhand von Beispielen die Herstellung und die Wirkweise der pharmazeutischen Zusammensetzung gemäß der vorliegenden Erfindung dargelegt. Diese Beispiele sollen lediglich exemplarisch sein und die Erfindung in keiner Weise beschränken.

Zur Herstellung einer pharmazeutischen Zusammensetzung in Form einer Injektionslösung wird zunächst in 30 ml einer 0,9%-igen NaCl-Lösung 1 ml einer gesättigten Lösung des Sicarius-Peptidtoxins HT1 in Lachesis D6 DHU (Deutsche Homöopathische Union) eingebracht. Anschließend gibt man 1 ml einer gesättigten Sicarius-Peptidtoxin-HT2-Lösung in Lachesis D6 DHU zu. Bevorzugt werden beide Schritte bei Raumtemperatur durchgeführt.

Dieser Mischcocktail wird durch Zugabe einer Substanz von Latrodectus geometricus und/oder Latrodectus obscurior vervollständigt. Dabei können auch die Gifte verschiedener Populationen von Latrodectus geometricus und/oder Latrodectus obscurior verwendet werden.

Anschließend wird die resultierende Mischung 10 mal zum Erdmittelpunkt hin geschüttelt.

Die entstehende Zusammensetzung kann bei etwa 7°C unter Ausschluss von Licht etwa 9 Monate aufbewahrt werden. Der experimentell bestimmte (elektrophoretisch) Wirkungsverlust beträgt nach 9 Monaten etwa 10%.

### Vergleichsbeispiel 1

In einer Zellkulturflasche (1250 ml) wird die Wirksamkeit der unterschiedlichen Giftextrakte anhand einer Zellauszählung nach 24, 48 und 100 Stunden, wie in der Tabelle 1 unten dargelegt, bestimmt. Dabei ist ersichtlich, dass die Population der Zellen nur unwesentlich abnimmt. Eine Abnahme der Zellpopulation in vitro entspricht einer Verringerung des Tumors in vivo.

| Blut-Hirnschranke-Durchdringungssubstanz in im DE 10 2005 027 665 beschriebenen Lösungsmittel | Hirn-Tumor zerstörende Substanz immer auch mit Androctonus bicolor | Spinnengiftkombi-Substanz-Ergebnis anhand einer Zellauszählung nach | | |
|---|---|---|---|---|
| | | 24 | 48 | 100h |
| Latrodectus bishopi | Sicarius sp. / Varanus sp. | ca. 80% | 85% | 94% |
| Latrodectus curacaviensis | Sicarius sp. / Varanus sp. | ca. 50% | 70% | 80% |
| Latrodectus dahli | Sicarius sp. / Varanus sp. | ca. 40% | 42% | 43% |
| Latrodectus erythromelas | Sicarius sp. / Varanus sp. | ca. 52% | 64% | 93% |
| Latrodectus hasselti | Sicarius sp. / Varanus sp. | ca. 49% | 72% | 85% |
| Latrodectus katipo | - - | | | |
| Latrodectus menavodi | - - | | | |
| Latrodectus rhodesiensis | - - | | | |
| Latrodectus revivensis | Sicariussp. / Varanus sp. | ca. 70% | 71% | 70% |
| Latrodectus schuchi | Sicariussp. / Varanus sp. | ca. 80% | 90% | 94% |
| Latrodectus tredecimguttatus | Sicariussp. / Varanus sp. | ca. 95% | 95% | 99% |
| Latrodectus variolus | Sicariussp. / Varanus sp. | ca. 83% | 88% | 91% |
| Latrodectus hesperus | Sicariussp. / Varanus sp. | ca. 24% | 20% | 20% |
| Latrodectus corallinus | Sicariussp. / Varanus sp. | ca. 31% | 17% | 11% |

### Beispiel 1

Analog obigem Vergleichsbeispiel 1 wurden die Zusammensetzungen der vorliegenden Erfindung, wie sie in Tabelle 2 unten dargelegt sind, untersucht.

| Hirntumorzerstörende Substanz ohne Andoctonus bicolor | Blut-Hirn-Schranken-Durchringungssubstanz | Zellauszählung | | |
|---|---|---|---|---|
| | | 24 | 48 | 100h |
| | | | | |
| Sicarius oweni | Latrodectus geometricus RSA | 40 | 40 | 27% |
| Sicarius testaceus | | 39 | 30 | 12% |
| Sicarius hahni | | 34 | 31 | 30% |
| Sicarius albospinosus | | 51 | 50 | 53% |
| Sicarius argentinensis | | 49 | 44 | 46% |
| Sicarius brasiliensis | | 42 | 40 | 28% |
| Sicarius terrosus | | 51 | 23 | 8% |
| Sicarius terrosus formae | | 58 | 18 | 3% |
| | | | | |
| Sicarius oweni | Latrodectus obscurior | 45 | 40 | 29% |
| Sicarius testaceus | Madagaskar | 51 | 50 | 45% |
| Sicarius argentinensis, | | 38 | 40 | 39% |
| Sicarius terrosus | | 62 | 51 | 48% |
| Sicarius terrosus formae | | 53 | 50 | 11% |
| | | | | |
| Sicarius oweni | Latrodectus geometricus | 41 | 32 | 30% |
| Sicarius testaceus | Südamerika | 37 | 37 | 38% |
| Sicarius argentinensis | | 44 | 40 | 31% |
| Sicarius terrosus | | 49 | 40 | 18% |
| Sicarius terrosus formae | | 59 | 32 | 9% |

Es zeigt sich, dass nach zunehmender Inkubation der Zellen kaum noch Zellwachstum stattfindet. In der Regel tötet das Sicarius-Toxin die Tumorzellen zunehmend ab. Als Durchdringungsenzym wird die Phospholipase. von Sicarius argentinensis verwendet. Diese Durchdringungssubstanz steht in ausreichender Menge zur Verfügung und erleichtert die Diffusion der Peptidtoxine wie beispielsweise des Sicarius-Toxins in das Gewebe und die Zellen.

### Therapiebeispiele

Zur Therapie von Hirntumoren wird den Patienten alle zwei oder drei Tage eine subkutane Injektion von 2-4 ml einer erfindungsgemäßen Zusammensetzung, je nach Schwere der Erkrankung, verabreicht. Der Therapieverlauf wird mittels Positronenemissionstomographie (PET) und über den Tumormarker LSA (lipidgebundene Sialinsäure) überwacht. Entsprechend kann die Therapie wenn nötig angepasst werden.

Zusätzlich werden täglich bis zu 20 Tabletten H15 oder 3 Kapseln Weihrauchblätter eingenommen, um eine Ödembildung, welche auch bei zu schneller Tumorzerstörung entstehen kann, zu unterbinden.

In den Kapseln, die Weihrauchblätter enthalten, sind daneben bevorzugt trockene Extrakte von Bambusa ventricosa enthalten.

### Behandlungsverlauf anhand verschiedener Patientenbeispiele

- Patientin, geboren 1999, Glioblastom diagnostiziert im 1. Lebensjahr. Inoperabel, nur 6 Bestrahlungen bis Juli 2000 erhalten. Im Juni 2001 Start mit einer Therapie mit der Zusammensetzung gemäß der vorliegenden Erfindung, zunächst täglich 5 Tage lang 3 ml, dann bis September 2005 jeden 2. Tag 2,5 ml, ab Oktober 2005 alle 3 Tage. Seit 2002 Februar geht das Mädchen wieder in den Kindergarten und fühlt sich gut. Der LSA "startete" bei 27 und hat sich seit November 2001 auf etwa 23 eingependelt. 1 mal pro Woche erhält das Kind 2 Weihrauchkapseln, um starker Ödembildung vorzubeugen. Das PET ist seit 2003 unauffällig. Die letzten vermerkten starken Kopfschmerzen waren im Dezember 2002.
- Patient, geboren 1958, Astrozytom Grad III, diagnostiziert im Mai 2000. Kam im Juni 2000 zu einer Münchner Ärztin, nach seiner 1. Operation. Nahm nach der Operation 15 Bestrahlungen und begann mit Caelix-Chemotherapie, welche er nur schlecht vertrug. Im Computertomogramm zeigte sich bis September 2000 eine starke Ausbreitung des Tumors diffus und vom Zelltyp hin zum Glioblastom. Im Oktober 2000 wollte der Patient mit einer Spinnengifttherapie beginnen. Er bekann zunächst mit einer täglichen Dosis von 4 ml vom 3.-7. Oktober 2000. Dann wurde bis zum 2. Januar 2002 täglich 3 mal injiziert. Ab 4. Januar bis Dezember 2006 alle 2 Tage 2,5 ml. Das Allgemeinbefinden des Patienten ist gut, lt. PET ist noch ein ca. 0,7 cm großer, schwach aktiver Tumorherd rechtsseitig im September 2006 gegeben. Der LSA war nach der Operation etwa 31, Ende November 2000 bei 25 und hat sich seit 2004 bei ungefähr 20,5 eingependelt. Der Patient nimmt täglich seit 14. Januar 2001 12 Tabletten H15.
- Patient geboren 1943, im Oktober 2002 Glioblastom diagnostiziert. Patient verweigert Operation, da er kaum auf Besserung hofft. Nimmt bis heute täglich 10 Tabletten H15 und jeden 2. Tag 1,5 ml der erfindungsgemäßen Zusammensetzung. Das PET ist seit der Aufnahme vom Oktober 2004 unauffällig! Der LSA startete 2003 mit 26,2, ist nun bei ca. 19.
- Patient, männlich, beginnt im Alter von 3 Jahren schlecht zu sehen. Mit 6 Jahren wird ein Hirntumor auf der Sehnervkreuzung diagnostiziert. Eine sich anschließende Strahlentherapie verläuft nicht positiv. Nach einer Erhöhung des Hirndrucks werden diverse Sonden, sogenannte Shunts, gelegt, um den Hirndruck zu reduzieren. Im Alter von knapp 9 Jahren wird dem Patienten eine Lebenszeit von wenigen Wochen prognostiziert. Die Behandlung mittels der pharmazeutischen Zusammensetzung gemäß der vorliegenden Erfindung beginnt mit einer Gabe von 2 ml der Zusammensetzung intramuskulär. Nach einer ersten Pause von 6 Tagen wird mit einem Abstand von 2 Tagen jeweils 1 ml der Zusammensetzung intramuskulär verabreicht. Im Laufe von 2 Wochen steigt die Dosis langsam auf 2 ml an. Einen Monat nach Beginn der Therapie konnte in einer Computertomogrammaufnahme ein leichtes Schrumpfen des Tumors diagnostiziert werden. Nach etwa einem Jahr war ein deutliches Schrumpfen des Tumors sichtbar. Die Dosierung wurde nun von 2 auf 3 ml erhöht und der Abstand der Gabe von 2 auf 3 Tagen ausgeweitet. Mit nunmehr 13 Jahren ist der Patient in einem Zustand, der ihm erlaubt, am regelmäßigen Leben teilzunehmen.

### Beispiel 2: Allgemeine Darstellung eines säulenchromatographischen Trennverfahrens

Zur Auftrennung der wirksamen Peptidtoxinfraktionen aus den Gesamtgiftcocktails von Spinnen der Gattungen Sicarius und Latrodectus sowie aus den Nattern der Gattung Elaphe können säulenchromatographische Trennverfahren bevorzug eingesetzt werden. Nachfolgend wird eine allgemeine Darstellung eines erfindungsgemäß anwendbaren säulenchromatographischen Trennverfahrens gegeben. Selbstverständlich kann ein Fachmann auch andere Verfahren zur Auftrennung von Peptidgemischen einsetzen. Die einzelnen, durch das Trennverfahren enthaltenen Fraktionen werden auf ihre Fähigkeit, Tumorzellen zu zerstören, entweder auf Zellkulturen oder im Tierexperiment überprüft. Die Verwendbarkeit einzelner Fraktionen aus dem Latrodectus-Gesamtgiftcocktail als Blut-Hirn-Schranke überwindendes Argens erfolgt bevorzugt im Tierversuchsmodell.

Allgemeine Darstellung eines säulenchromatographischen Trennverfahrens:

Zur Trennung in die einzelnen Bestandteile/Fraktionen wird das Gesamtgift in 5 mL Proteinlösungsmittel für Säulenchromatografie, welches sich aus 0,25 M Tris/HCl, pH 6,5 bis 7,3, 1,92 M Glycin in destilliertem und deionisiertem Wasser zusammensetzt, aufgenommen. Es wird mit dem Gesamtgiftcocktail eine gesättigte Lösung in einem Teflonvial hergestellt. Zur homogenen Durchmischung des Gesamtgiftcocktails mit dem Proteinlösungsmittel wird 60 Sekunden unter Vermeidung von Schaumbildung auf dem Vortex geschüttelt.

Nach der Homogenisierung wird die Lösung über einen Plexiglastrichter in eine transparente und stehende Plexiglassäule, die einen Innendurchmesser von 1 cm, eine Wandstärke von 2 mm und eine Höhe von 50 cm aufweist und unten konisch bis auf ca. 1,5 mm zuläuft, aber offen ist, eingefüllt. In der Säule ist 15 mL Gel (ACA 34; Matrix 3 % Acrylamid 4 % Agarose; Fraktionierungsbereich (MW): Proteine von 20 - 350 kDa, Ausschlußgrenze: 750 kDa, Kügelchendurchmesser: 60 - 140 µm). Die eingebrachte Giftlösung verdrängt beim eindringen in das Gel die im Gel befindliche Pufferlösung. Nach vollständigem Eindringen der Giftlösung in das Gel wird portionsweise (ohne das Gel trocken laufen zu lassen) 165 mL Lösungsmittel (0,25 M Tris/HCl, pH 6,5 - 7,3, 1,92 M Glycin) auf die Säule gebracht. Dieses Lösungsmittel verdrängt bei seinem Durchlauf durch das Gel die darin befindliche Giftlösung. Die ersten 15 mL, die unten aus der Säule tropfen sind restlicher Gelpuffer und werden verworfen. Anschließend werden 40 Fraktionen zu 4 mL aufgefangen. Durch die chemischen und physikalischen Eigenschaften dieses Trennsystemes bedingt ergaben sich die 4 mL für die einzelnen Fraktionen. Daß auch wirklich nur eine Komponente pro Fraktion zu finden ist wird mit SDS-PAGE überprüft. Nur eine Bande pro Fraktion.

Für die SDS-PAGE wird als Auftragspuffer für die Fraktion aufs Gel zum Peptidbindungs- und Proteinschutz Roti Load 1 + 2 (Carl Roth GmbH & Co KG, Karlsruhe:SDS-,Glycerol- ,Bromphenolblau, Phosphatpuffer, Roti Load 1 mit Mercaptoethanol, Roti Load 2 ohne Mercaptoethanol) verwendet.

Die einzelnen Fraktionen nach der Säulentrennung werden in sauberen, zuvor sterilisierten und verschraubbaren Teflonvials getrennt aufgefangen.

### Beispiel 3: Herstellung der Sicarius-Peptidtoxine

Die Sicarius-Spinnen werden, wie vorstehend näher beschrieben, gemolken, um den Gesamtgiftcocktail zu gewinnen. Das Gesamtgift wird dann über säulenchromatographische Verfahren, beispielsweise über HPLC, gewonnen. Die Fraktionen 4, 8, 11 und 14 enthalten die hirntumorzerstörenden Substanzen bei den afrikanischen Sicarius-Arten oweni, testaceus, hahni, albospinosus. Die Fraktionen 5 und 10 enthalten die himtumorzerstörenden Substanzen bei den südamerikanischen Sicarius-Arten argentinensis, brasiliensis, terrosus und terrosus formae.

Mit einer SDS-PAG-Elekrophorese ergaben sich mit Coomassie-Blau-Färbung folgende (gemittelte) Molekulargewichte der gefriergetrockneten Substanzen:

### afrik. Sicarius:

| | |
|---|---|
| Fraktion 4 | 55 000 Da |
| Fraktion 8 | 70 000 Da |
| Fraktion 11 | 82 000 Da |
| Fraktion 14 | 85 000 Da |

### südamerik. Sicarius:

| | |
|---|---|
| Fraktion 5 | 25 000 Da |
| Fraktion 10 | 81 000 Da |

Die Fraktion 4 entspricht dem HT1-Peptidtoxin, die Fraktion 10 dem HT2-Peptidtoxin.

Die Peptidtoxine aus Latrodectus und Elaphe wurden wie HT1 und HT2 auf käuflichen Standard-Glioblastom und Astrozytom-Zellkulturen ausgetestet und zeigten ebenfalls den gewünschten Effekt.

Die Phospholipase aus dem Gesamtgiftcocktail von Sicarius argentinensis wurde auf gleiche Weise gewonnen und bestimmt, wie die Peptidtoxine. Ihr Molekulargewicht beträgt 38 000, womit sie sich von anderen Sicariussubstanzen abgrenzen lässt. Durch eine enzymkinetische Untersuchungsreihe konnte darüber hinaus die Zuordnung zum Phospholipasetyp bestätigt werden.

## Patentansprüche

1. Pharmazeutische Zusammensetzung zur Behandlung eines Hirntumors, enthaltend in einer pharmazeutisch wirksamen Menge
a) Peptidtoxin aus Spinnen der Gattung Sicarius und/oder Mundsekret von Nattern der Gattung Elaphe; und
b) Gesamtgift oder Peptidtoxine von Spinnen der Gattung Latrodectus mit einem Molekulargewicht von 5 kDa bis 130 kDa und der Fähigkeit zur Überwindung der Blut-Hirn-Schranke, jeweils keine Latrotoxine enthaltend.

2. Pharmazeutische Zusammensetzung gemäß Anspruch 1, **dadurch gekennzeichnet, dass** sie zusätzlich inaktivierten Parapoxvirus ovis, vorzugsweise Stamm D1701, enthält.

3. Pharmazeutische Zusammensetzung gemäß einem oder mehreren der vorangegangenen Ansprüche, **dadurch gekennzeichnet, dass** die Spinnen der Gattung Sicarius ausgewählt sind aus der Gruppe, bestehend aus den Arten Sicarius oweni, Sicarius testaceus, Sicarius hahni, Sicarius albospinosus, Sicarius argentinensis, Sicarius brasiliensis, Sicarius terrosus und Sicarius terrosus formae, wobei die Arten Sicarius terrosus und Sicarius terrosus formae insbesondere bevorzugt sind.

4. Pharmazeutische Zusammensetzung gemäß einem oder mehreren der vorangegangenen Ansprüche, **dadurch gekennzeichnet, dass** das Peptidtoxid aus Spinnen der Gattung Sicarius das HT1- und/oder das HT2-Peptidtoxin ist.

5. Pharmazeutische Zusammensetzung gemäß einem oder mehreren der vorangegangenen Ansprüche, **dadurch gekennzeichnet, dass** sie zusätzlich Lachesis D6 enthält.

6. Pharmazeutische Zusammensetzung gemäß einem oder mehreren der vorangegangenen Ansprüche, **dadurch gekennzeichnet, dass** die Spinnen der Gattung Latrodectus ausgewählt sind aus der Gruppe, bestehend aus den Arten Latrodectus bishopi, Latrodectus curacaviensis, Latrodectus dahli, Latrodectus erythromelas, Latrodectus hasselti, Latrodectus katipo, Latrodectus menavodi, Latrodectus rhodesiensis, Latrodectus revivensis, Latrodectus schuchi, Latrodectus tredecimguttatus, Latrodectus variolus, Latrodectus hesperus, Latrodectus corallinus, Latrodectus geometricus und Latrodectus obscurior.

7. Pharmazeutische Zusammensetzung gemäß einem oder mehreren der vorangegangenen Ansprüche, **dadurch gekennzeichnet, dass** die Spinnen der Gattung Latrodectus bevorzugt die Arten Latrodectus geometricus und Latrodectus obscurior sind.

8. Pharmazeutische Zusammensetzung gemäß einem oder mehreren der vorangegangenen Ansprüche, **dadurch gekennzeichnet, dass** sie weiterhin Phospholipasen aus Sicarius enthält.

9. Zusammensetzung gemäß einem der Ansprüche 1-8 zur Verwendung in einem Verfahren zur Behandlung von Hirntumoren und Glioblastomen.

10. Verfahren zum Herstellen einer Zusammensetzung gemäß einem der Ansprüche 1-8 mit den Schritten:
- Bereitstellen einer isotonischen Natriumchloridlösung mit Sicarius-Peptidtoxin;
- wahlweise Zugabe eines weiteren Sicarius-Peptidtoxins zu der Natriumchloridlösung; und
- Zugabe eines Gesamtgiftes oder von Peptidtoxinen von Spinnen der Gattung Latrodectus, mit einem Molekulargewicht von 5 kDa bis 130 kDa und der Fähigkeit zur Durchdringung der Blut-Hirn-Schranke, jeweils keine Latrotoxine enthaltend;
- Vermischen der Bestandteile, insbesondere durch Schütteln der Mischung.

11. Verfahren gemäß Anspruch 10, wobei zusätzlich vor dem Schütteln ein Immunmodulator, bevorzugt inaktiviertes Parapoxvirus ovis, zugegeben wird.

12. Verfahren gemäß Anspruch 10 oder 11, wobei mindestens eines der Sicarius-Peptidtoxine in Lachesis D6 gelöst sind.

13. Verfahren gemäß einem oder mehreren der Ansprüche 10-12, wobei das erste Sicarius-Peptidtoxin ein Sicarius-Peptidtoxin HT1 ist.

14. Verfahren gemäß einem oder mehreren der Ansprüche 10-13, wobei das zweite Sicarius-Peptidtoxin ein Sicarius-Peptidtoxin HT2 ist.

15. Kit of parts zur Behandlung von Hirntumoren und Glioblastomen umfassend
i) eine pharmazeutische Zusammensetzung gemäß einem oder mehreren der Ansprüche 1-8; und
ii) Extrakt aus Weihrauch und/oder Bambus.

## Claims

1. Pharmaceutical composition for treating a brain tumor, which contains in a pharmaceutical effective quantity:
a.) peptide toxin of spiders of genus Sicarius and / or the oral secretion of vipers of genus Elaphe; and
b.) total poison or peptide toxins of spiders of genus Latrodectus with a molecular weight of 5 kDa to 130 kDa and the ability to overcome the blood-brain barrier, each time without Latrotoxine content.

2. Pharmaceutical composition according to claim 1, **characterized in that** it additionally contains inactivated Parapoxvirus Ovis, preferably strain D1701.

3. Pharmaceutical composition according to one or more of the previous claims, **characterized in that** the spiders of genus Sicarius are selected from the group, consisting of the species Sicarius oweni, Sicarius testaceus, Sicarius hahni, Sicarius albospinosus, Sicarius argentinensis, Sicarius brasiliensis, Sicarius terrosus and Sicarius terrosus formae, wherein the species Sicarius terrosus and Sicarius terrosus formae are particularly preferred.

4. Pharmaceutical composition according to one or more of the previous claims, **characterized in that** the peptide toxin from spiders of genus Sicarius is peptide toxin HT1 and/or HT2.

5. Pharmaceutical composition according to one or more of the previous claims, **characterized in that** it additionally contains Lachesis D6.

6. Pharmaceutical composition according to one or more of the previous claims, **characterized in that** the spiders of genus Latrodectus are selected from the group, consisting of the species Latrodectus bishopi, Latrodectus curacaviensis, Latrodectus dahli, Latrodectus erythromelas, Latrodectus hasselti, Latrodectus katipo, Latrodectus menavodi, Latrodectus rhodesiensis, Latrodectus revivensis, Latrodectus schuchi, Latrodectus tredecimguttatus, Latrodectus variolus, Latrodectus hesperus, Latrodectus corallinus, Latrodectus geometricus and Latrodectus obscurior.

7. Pharmaceutical composition according to one or more of the previous claims, **characterized in that** the spiders of genus Latrodectus are preferably the specied Latrodectus geometricus and Latrodectus obscurior.

8. Pharmaceutical composition according to one or more of the previous claims, **characterized in that** it additionally contains phospholipases from Sicarius.

9. Composition according to one of claims 1-8 for use in a method of treating brain tumors and glioblastoma.

10. Method for producing a composition according to one of claims 1-8 with the steps:
- preparing an isotonic solution of sodium chloride with peptide toxin from Sicarius;
- optionally adding a further peptide toxin from Sicarius to the solution of sodium chloride; and
- adding a total poison or peptide toxins of spiders of genus Latrodectus with a molecular weight of 5 kDa to 130 kDa and the ability to overcome the blood-brain barrier, each time without Latrotoxine content;
- mixing the components, especially by shaking the composition.

11. Method according to claim 10, wherein additionally before shaking an immunomodulator, preferably inactivated Parapoxvirus Ovis, is added.

12. Method according to claim 10 or 11, wherein at least one of the peptide toxins of Sicarius is dissolved in Lachesis D6.

13. Method according to one or more of claims 10-12, wherein the first peptide toxin from Sicarius is a Sicarius peptide toxin HT1.

14. Method according to one or more of claims 10-13, wherein the second peptide toxin from Sicarius is a Sicarius peptide toxin HT2.

15. Kit of parts for treating of brain tumors and glioblastoma, comprising:
i) a pharmaceutical composition according to one or more of claims 1-8; and
ii) extract of incense and / or bamboo.

## Revendications

1. Composition pharmaceutique destinée à traiter une tumeur du cerveau, contenant en quantité pharmaceutiquement efficace
a) une toxine peptidique provenant d'araignées du genre Sicarius et/ou une sécrétion buccale provenant de couleuvres du genre Elaphe et
b) du venin complet ou des toxines peptidiques qui proviennent d'araignées de l'espèce Latrodectus, dont le poids moléculaire est compris entre 5 kDa et 130 kDa et qui sont capables de franchir la barrière sang-cerveau mais ne contiennent pas de latrotoxine.

2. Composition pharmaceutique selon la revendication 1, **caractérisée en ce qu'**elle contient en outre Parapoxvirus ovis inactivé, de préférence de souche D 1701.

3. Composition pharmaceutique selon une ou plusieurs des revendications précédentes, **caractérisée en ce que** les araignées du genre Sicarius sont choisies parmi le groupe formé par les espèces Sicarius oweni, Sicarius testaceus, Sicarius hahni, Sicarius albospinosus, Sicarius argentinensis, Sicarius brasiliensis, Sicarius terrosus et Sicarius terrosus formae, parmi lesquelles les espèces Sicarius terrosus et Sicarius terrosus formae sont particulièrement recommandées.

4. Composition pharmaceutique selon une ou plusieurs des revendications précédentes, **caractérisée en ce que** la toxine peptidique provenant des araignées du genre Sicarius est la toxine peptidique HT 1 et/ou HT 2.

5. Composition pharmaceutique selon une ou plusieurs des revendications précédentes, **caractérisée en ce qu'**elle contient en outre Lachesis D 6.

6. Composition pharmaceutique selon une ou plusieurs des revendications précédentes, **caractérisée en ce que** les araignées du genre Latrodectus sont choisies parmi le groupe formé par les espèces Latrodectus bishopi, Latrodectus curacaviensis, Latrodectus dahli, Latrodectus erythromelas, Latrodectus hasselti, Latrodectus katipo, Latrodectus menavodi, Latrodectus rhodesiensis, Latrodectus revivensis, Latrodectus schuchi, Latrodectus tredecimguttatus, Latrodectus variolus, Latrodectus hesperus, Latrodectus corallinus, Latrodectus geometricus et Latrodectus obscurior.

7. Composition pharmaceutique selon une ou plusieurs des revendications précédentes, **caractérisée en ce que** les araignées du genre Latrodectus sont de préférence celles des espèces Latrodectus geometricus et Latrodectus obscurior.

8. Composition pharmaceutique selon une ou plusieurs des revendications précédentes, **caractérisée en ce qu'**elle contient en outre des phospholipases provenant de Sicarius.

9. Composition selon une des revendications 1 à 8, à utiliser dans un procédé pour traiter les tumeurs du cerveau et les glioblastomes.

10. Procédé pour produire une composition selon une des revendications 1 à 8, comprenant les étapes suivantes :
- fournir une solution isotonique de chlorure de sodium contenant une toxine peptidique de Sicarius,
- ajouter éventuellement une autre toxine peptidique de Sicarius à la solution de chlorure de sodium et
- ajouter un venin complet ou des toxines peptidiques qui proviennent d'araignées du genre Latrodectus, dont le poids moléculaire est compris entre 5 kDa et 130 kDa et qui sont capables de franchir la barrière sang-cerveau mais ne contiennent pas de latrotoxine,
- mélanger les composants, en particulier en agitant le mélange.

11. Procédé selon la revendication 10, d'après lequel on ajoute avant l'agitation un immunomodulateur, de préférence Parapoxvirus ovis inactivé.

12. Procédé selon la revendication 10 ou 11, d'après lequel au moins une toxine peptidique de Sicarius est dissoute dans Lachesis D 6.

13. Procédé selon une ou plusieurs des revendications 10 à 12, d'après lequel la première toxine peptidique de Sicarius est une toxine peptidique HT 1 de Sicarius.

14. Procédé selon une ou plusieurs des revendications 10 à 13, d'après lequel la deuxième toxine peptidique de Sicarius est une toxine peptidique HT 2 de Sicarius.

15. Trousse d'éléments pour traiter des tumeurs du cerveau et des glioblastomes, comprenant :
i) une composition pharmaceutique selon une ou plusieurs des revendications 1 à 8 et
ii) un extrait d'encens et/ou de bambou.
